# EUROPEAN PATENT SPECIFICATION

(11) **EP 3 253 892 B1**
(45) Date of publication and mention of the grant of the patent: **13.11.2019**
(21) Application number: 16746901.4
(22) Date of filing: 20.01.2016
(51) Int. Cl.: C13K 1/02, C12P 7/10, C12P 19/02, D21C 1/04

(54) **CONTROLLING THE PRETREATMENT OF A SOFTWOOD STARTING MATERIAL**
STEUERUNG DER VORBEHANDLUNG EINES WEICHHOLZAUSGANGSMATERIALS
CONTRÔLE DU PRÉTRAITEMENT DE BOIS TENDRE COMME MATIÈRE DE DEPART

(30) Priority: 02.02.2015 SE 1550104
(43) Date of publication of application: 13.12.2017
(73) Proprietor: Sekab E-Technology AB, 891 26 Örnsköldsvik (SE)
(72) Inventor: HÄGGLUND, Karin, 892 92 Domsjö (SE)
(74) Representative: Kransell & Wennborg KB
(86) International application number: PCT/SE2016/050034
(87) International publication number: WO 2016/126185

(56) References cited:
- EP-A1- 2 540 833
- WO-A1-2012/066042
- US-A- 4 237 226
- US-A1- 2014 083 939
- MONAVARI ET AL: "Improved one-step steam pretreatment of SO2-impregnated softwood with time-dependent temperature profile for ethanol production", BIOTECHNOLOGY PROGRESS, vol. 26, 2010, pages 1054-1060, XP002783554,
- SÖDERSTRÖM ET AL: "Two-step steam pretreatment of softwood with SO2 impregnation for ethanol production", APPLIED BIOCHEMISTRY AND BIOTECHNOLOGY, vol. 98-100, 2002, pages 5-21, XP002783555,
- TUCKER M P ET AL.: 'Fourier Transform Infrared Quantification of Sugars in Pretreated Biomass Liquors' APPLIED BIOCHEMISTRY AND BIOTECHNOLOGY vol. 84 - 86, no. 1-9, 01 January 2000, pages 39 - 50, XP055199837 DOI: 10.1385/ABAB:84-86:1-9:39
- BOONTIAN N: 'Conditions of the Anaerobic Digestion of Biomass' INTERNATIONAL JOURNAL OF BIOLOGICAL, BIOMOLECULAR, AGRICULTURAL, FOOD AND BIOTECHNOLOGICAL ENGINEERING vol. 8, no. 9, 01 January 2014, pages 1036 - 1040, XP055474931
- GONZALEZ-MUNOZ M J: 'Production of hemicellulosic sugars from wood by sequential steps of aqueous extraction and acid hydrolysis' WOOD SCIENCE AND TECHNOLOGY vol. 46, no. 1-3, 24 February 2012, pages 271 - 285, XP019999194 DOI: 10.1007/S00226-011-0408-0

## Description

### Field of the present invention

The present invention relates to a method for pretreating a softwood starting material. In particular the invention relates to control of processes of pretreatment of a soft wood biomass to improve the digestibility of the biomass, wherein the concentration of glucose and the concentration of mannose are measured and the ratio is used for controlling at least one process parameter.

### Background

Biorefineries producing green chemicals and fuels from renewable resources offer an alternative to oil refineries based on dwindling supplies of petroleum and permit a move towards improved energy security. Lignocellulosic residues from forestry are attractive as feedstocks, since they are abundant, relatively inexpensive, and are not used for food. Lignocellulose consists mainly of lignin and two classes of polysaccharides, cellulose and hemicellulose. The polysaccharides can be hydrolyzed to sugars which can be used as a platform chemical for the production of a host of other chemicals, for example by fermentation of the sugars in to various fermentation products, such as bioalcohols. The fermentation processes utilizes fermenting agents to convert the sugar in to fermentation products. One such fermenting agent is baker's yeast (*Saccharomyces cerevisiae*) which can be used to ferment sugars in to ethanol. The hydrolysis of cellulose is typically preceded by a pretreatment, in which the cellulose is made more digestibility, such as more accessible to cellulolytic enzymes.

WO 2012/066042 refers to NIR measurements in the production of a target chemical from cellulose, whereby the slurry is analysed with NIR spectroscopy.

Söderström et al, Applied Biochemistry and Biotechnology; 2002, pp. 5-21, refer to stem pretreatment of softwood with SO₂ impregnation and discloses i.a. the yield of glucose and mannose as a function of the severity of the pretreatment.

### Summary of the present invention

To render the cellulose more accessible for the enzymes in a subsequent enzymatic hydrolysis step, it is important that the pretreatement is severe enough. However, too harsh pretreatment leads to degradation of sugars and increased levels of substances which are toxic to the hydrolytic enzymes and to fermenting organisms. Examples of such toxic substances include furfural, hydroxymethylfurfural, acetic acids and phenolic compounds. An accurately controlled pretreatment step is therefore important for the yield in subsequent process steps, such as for example enzymatic hydrolysis and fermentation. The present inventor has surprisingly discovered that a calculated ratio between glucose and mannose in a slurry of pretreated soft wood can be used to determine if the pretreatment process has been too harsh or to mild. Based on this discovery the inventor has further realized that a process of pretreating softwood can be controlled by measuring the ratio glucose : mannose, comparing the ratio to a reference value and controlling at least one process parameter of the pretreatment process in response to a difference between the measured ratio and the reference value.

Thus, a first aspect of the present invention relates to a method for pretreating a softwood starting material comprising the following steps:
a) pretreating the softwood starting material in a pretreatment process to form a slurry;
b) measuring the concentration of glucose and the concentration of mannose in the slurry formed in step a);
c) calculating a ratio glucose : mannose from the concentrations measured in step b)
d) comparing the ratio calculated in step c) to a reference value and if the ratio calculated in step c) differs from the reference value, controlling at least one process parameter of the pretreatment process in response to the difference.

### Brief description of the figure

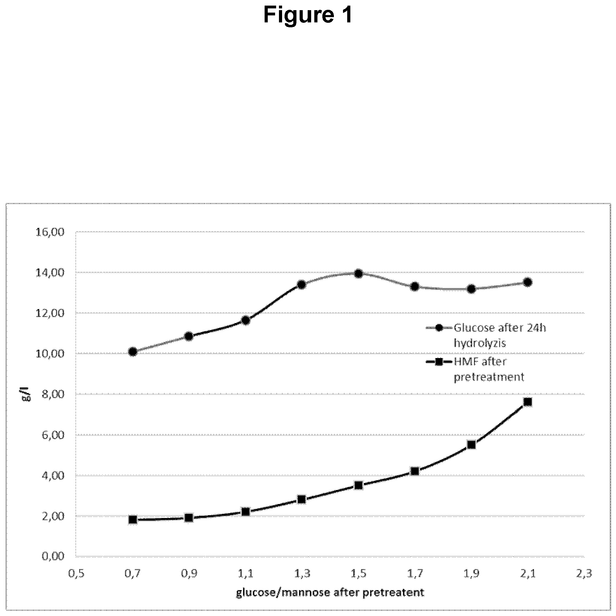

Figure 1 shows the result from an experiment where softwood chips were pretreated at several different severities in a continuous pretreatment reactor. Glucose, mannose and HMF concentrations were measured in the different pretreated slurries obtained by the pretreatment. Each of the pretreated slurries were thereafter subjected to enzymatic hydrolysis and the glucose concentrations in the obtained enzymatic hydrolysates were measured. Figure 1 shows two curves. In the lower curve the HMF concentration in the pretreated slurries were plotted as a function of the glucose:mannose ratios in the pretreated slurries. In the upper curve, the concentrations of glucose in the obtained enzymatic hydrolysates were plotted as a function of the glucose:mannose ratios in the pretreated slurries. The Y axis shows the glucose concentration in the enzymatic hydrolysates (in grams/liter), respectively the HMF concentrations in the pretreated slurries (in grams/liter). The x-axis shows the weight ratios of glucose:mannose in the pretreated slurries.

### Detailed description

An effective pretreatment is needed to liberate the cellulose in a lignocellulosic starting material from the lignin seal and its crystalline structure so as to render it accessible for a subsequent hydrolysis step. If the pretreatment is not harsh enough, it will be harder to hydrolyze the carbohydrates to sugars, and/or to digest it to biogas. However, if the pretreatment is too harsh, larger amounts of sugars will be degraded and fermentation inhibitors such as HMF, furan aldehydes (e.g. furfural), acetic acid and/or phenolic compounds will be formed, which in turn might impair hydrolysis and/or fermentation processes. Similarly, a too harsh pretreatment can also reduce the yield from a biogas digestion process. Efficient control of the pretreatment step is thus highly beneficial for processes for production of green chemicals from lignocellulosic biomass. The composition of a slurry of pretreated lignocellulosic biomass will differ depending on the severity of the pretreatment. For example the concentration of inhibitors and sugars will change in response to the severity of the pretreatment. The present inventor has realized that the concentration of different specific molecules in the pretreated slurry can be monitored in order to measure the harshness of the pretreatment. Surprisingly, the present inventor has demonstrated that, if the biomass is a softwood biomass, the ratio between glucose and mannose can be used to determine if the pretreatment process has been too harsh or to mild. The inventor has further demonstrated that the pretreatment process can be controlled by comparing the ratio to a reference value. If the ratio differs from the reference value, the pretreatment process can be controlled by adjusting at least one process parameter of the pretreatment process in response to the difference.

Given the fact that the concentration of a host of different molecules will change during the pretreatment step, and vary in concentration depending on the severity of the pretreatment step, it is highly surprising that the pretreatment step can be efficiently controlled simply by measuring the concentration of two specific molecules, i.e. glucose and mannose. Importantly, the present inventor has demonstrated that this method is suitable for some lignocellulosic substrates but not for other. In particular, the method works very well for controlling the pretreatment of a softwood starting material but does not appear to be suitable for controling the pretreatment of straw.

Thus, a first aspect of the present invention relates to a method for pretreating a softwood starting material comprising the following steps:
a) pretreating the softwood starting material in a pretreatment process to form a slurry;
b) measuring the concentration of glucose and the concentration of mannose in the slurry formed in step a);
c) calculating a ratio glucose : mannose from the concentrations measured in step b)
d) comparing the ratio calculated in step c) to a reference value and if the ratio calculated in step c) differs from the reference value, controlling at least one process parameter of the pretreatment process in response to the difference.

An accurate pretreatment is important for subsequent step such as production of green chemicals from the slurry. Production of such green chemicals can involve hydrolyzing the cellulose present in the slurry into sugars. Thus in one embodiment the method further comprise the step e1) hydrolyzing the slurry obtained in step a) such that a sugar solution is obtained. In a preferred embodiment the hydrolyzing in step e1) is enzymatic hydrolysis. The sugars obtained from the hydrolysis process can for example be fermented in to a target chemical. Thus, in one embodiment the method further comprising the step
f) fermenting the sugar solution obtained in step e1) using an fermenting agent such that a fermentation product is obtained. In one embodiment step e1) and step f) is performed simultaneously in an simultaneous saccharification and fermentation process (SSF). In another embodiment step e1) and step f) is performed separately in a separate hydrolysis and fermentation process (SHF). In one embodiment at least one endoglucanase, at least one exoglucanase and at least one β-glucosidase is added in step e1). In one embodiment the fermentation product is selected from alcohols, acids, alkanes, alkenes, aromatics, aldehydes, ketones, biopolymers, proteins, peptides, amino acids or vitamins. In a preferred embodiment the fermentation product is selected from ethanol, butanol, acetic acid, butyric acid and succinic acid. In one embodiment the fermenting agent is bacteria and/or yeast. In one embodiment the fermenting organism is yeasts from the genera Saccharomyces, Pichia or Candida. In one embodiment the fermenting organism is wild type, mutant or recombinant Saccharomyces cerevisiae. In another embodiment the fermenting organism is bacteria from the genera Zymomonas or Escherichia.

The pretreated slurry can also be used for anaerobic digestion of the slurry into biogas. The requirements of a pretreated slurry which shall be used as a substrate for biogas digestion is similar to the requirements of a pretreated slurry which shall be used for production of sugars by a hydrolytic process, such as an enzymatic hydrolysis process. Thus in one embodiment the method further comprising the step
e2) anaerobic digestion of the slurry obtained in step a) or of the sugar solution obtained in step e1) such that biogas is obtained.

The present inventor has discovered that a mild pretreatment gives rise to a lower glucose : mannose ratio than a more severe pretreatment. Furthermore, the inventor has discovered that if the starting biomass is softwood, it is possible to control the pretreatment process by analyzing the ratio in the pretreated slurry and comparing it to a reference value. If the value is lower than the reference value, process parameters can be adapted such that the severity of the pretreatment is increased. Conversely, If the ratio glucose : mannose is higher than the reference value, this is an indication of that the pretreatment has been too severe. Thus, the pretreatment can be controlled by adjusting process parameters such that the severity of the pretreatment is decreased.

Therefore, In one embodiment;
- if the ratio glucose : mannose is lower than the reference value at least one process parameter of the pretreatment process is controlled such that the severity of the pretreatment is increased, and
- if the ratio glucose : mannose is higher than the reference value at least one process parameter of the pretreatment process is controlled such that the severity of the pretreatment is decreased.

The present inventor has discovered that a ratio (w/w) of glucose : mannose in the pretreated slurry in the range 1:1 to 1.6:1 gives rise to a high yield of product produced in subsequent step. For example a high yield of sugars can be produced in a subsequent enzymatic hydrolysis step and a high yield of ethanol fermented from the sugars produced in the enzymatic hydrolysis step. The inventor has discovered that lower yields of fermentation product is obtained if the ratio (w/w) of glucose : mannose in the pretreated slurry fall outside of this defined range. Therefore, in one embodiment the ratio (w/w) glucose : mannose is calculated in step c) and the reference value is in the range 1:1 to 1.6:1 preferably 1.1:1 to 1.5:1.

As can be seen from figure 1, pretreatment conditions which is more severe than the conditions giving rise to a glucose:mannose ratio of about 1.5 does not give rise to any further increase in glucose concentration, in an enzymatically produced hydrolysate produced from the pretreated slurry. Furthermore, the concentration of HMF increase rapidly at glucose : mannose ratios above 1.5:1. The increasing HMF concentrations are a result of increasing glucose degradation which is negative for the yield of obtained glucose. Furthermore, the higher HMF levels formed at the more severe pretreatment conditions (i.e. at the higher glucose:mannose ratios) are negative for subsequent fermentation processes since HMF and other inhibiting degradation products will inhibit the fermenting agent such that the yield of fermentation product will decrease. The present inventor has discovered that the optimal glucose:mannose ratio for obtaining a high yield of a fermentation product, e.g. ethanol, produced from the obtained hydrolysate is in the range 1.2: 1 to 1.4 :1. Therefore, in one particularly preferred embodiment the reference value is in the range 1.2: 1 to 1.4 :1.

In one embodiment the reference value is an interval. In one embodiment the interval is 1:1 to 1.6:1 preferably 1.1:1 to 1.5:1 most preferably 1.2: 1 to 1.4 :1.

There are several advantages with using the glucose:mannose ratio for controlling the severity of the pretreatment of a soft wood biomass. First of all, the method is solid and by controlling the pretreatment, such that the ratio is kept within a predetermined interval, a high yield of product can be obtained. The present inventor has not found any other chemicals in the pretreated slurry which is as useful for controlling the pretreatment process or for predicting the yield from subsequent enzymatic hydrolysis or fermentation steps. Furthermore, the measurement of glucose and mannose is simple and accurate compared to several other chemicals. For example the concentration of HMF is harder to measure accurately and does not predict the obtained yield from subsequent enzymatic hydrolysis or fermentation steps as good as the glucose:mannose ratio, data not shown.

In the context of the present disclosure, "pretreating" refers to modifying a lignocellulosic starting material to make the cellulose therein more accessible to hydrolytic activity in a subsequent hydrolysis step. However, in some embodiments of the present disclosure, the pretreatment can be performed to make the lignocellulosic starting material more suitable to anaerobic digestion for the production of biogas. The person of skill in the art is well aware of various pretreatment methods that may be employed for that purpose. As an example, the pretreatment may be acidic pretreatment or alkali pretreatment. Thus, the pretreatment may be aided by the addition of a catalyst. The catalyst may be added as a fluid in an impregnation step, which is followed by a heating step. The heating is normally achieved by the addition of steam. The fluid may be an acidic solution, such as an aqueous mineral acid solution, such as sulfuric or sulfurous acid. The impregnation may also be performed with a gas, such as a SO₂ gas or CO₂ gas, or with the combination of a gas and a liquid. The pretreatment may also comprise steaming. Steaming refers to a process used to drive air out from the cellulosic biomass to facilitate further hydrolysis of the cellulose. Steaming is a well-known method for pretreating e.g. lignocellulosic biomass. As another example, the pretreatment may involve steam explosion. Steam explosion is a process that combines steam, rapid pressure release and hydrolysis for rupturing cellulosic fibers. Steam explosion may be performed with or without a preceding addition of a catalyst. Thus, in one embodiment the pretreatment is acidic pretreatment using an acidic catalyst or alkaline pretreatment using an alkaline catalyst. In one preferred embodiment the pretreatment is acidic pretreatment using an acidic catalyst. In one embodiment the acidic catalyst is an acid solution, such as a mineral acid solution or a gas such as a sulfur dioxide. In one embodiment the catalyst is selected from sulfuric acid, sulfurous acid and sulfuric acid. In one embodiment the pretreatment includes steam explosion. In one embodiment the pretreatment is a dilute acid pretreatment.

The most important severity parameters of the pretreatment process reaction is the pH, the temperature, the pressure and the residence time of the starting material in the pretreatment process. Therefore, in one embodiment the process parameter of the pretreatment process is controlled by:
- adjusting a temperature of the pretreatment step;
- adjusting a pressure of the pretreatment step
- adjusting a residence time of the pretreatment step and/or
- adjusting a pH of the pretreatment step

In one embodiment process parameter of the pretreatment process is controlled such that the severity of the pretreatment is increased by:
- increasing a temperature of the pretreatment step;
- increasing a pressure of the pretreatment step
- increasing a residence time of the pretreatment step and/or
- decreasing a pH of the pretreatment step if the pretreatment is acidic pretreatment or increasing a pH of the pretreatment step if the pretreatment is alkaline pretreatment

In one embodiment process parameter of the pretreatment process is controlled such that the severity of the pretreatment is decreased by:
- decreasing a temperature of the pretreatment step;
- decreasing a pressure of the pretreatment step
- decreasing a residence time of the pretreatment step and/or
- increasing a pH of the pretreatment step if the pretreatment is acidic pretreatment or decreasing a pH of the pretreatment step if the pretreatment is alkaline pretreatment

In one embodiment the temperature of the pretreatment in step a) is 190-225 °C, such as 200-220 °C.

In one embodiment the pH of the pretreatment in step a) is 1,2-2,2, preferably 1,2-2,0. In one embodiment the residence time in the pretreatment step is 3-10 min, such as 4-8 minutes.

The concentration of glucose and mannose can be measured using any method known in the art. HPLC based technique can for example be used in the process of measuring glucose and mannose concentration.

In one embodiment the softwood starting material is shaving or chips from softwood. In one embodiment the softwood starting material comprises spruce, pine and/or fir.

### Examples

Wood chips from spruce was pretreated at several different conditions using temperatures ranging between 200-220 °C; pH in the range 1.2-2.0 and residence times in the range 4-8 minutes in a continuous vertical pretreatment reactor followed by steam explosion. The HMF concentrations, the glucose concentrations and the mannoses concentration in the obtained pretreated slurries were measured using HPLC based methods. The pretreated slurries were thereafter subjected to enzymatic hydrolysis for 24 h using commercially available preparations of cellulase and cellobiase. The glucose concentrations in the obtained hydrolysates were thereafter measured using HPLC based methods. The weight ratios of glucose to mannose in the pretreated slurries were calculated. In figure 1 the glucose concentrations in the enzymatic hydrolyzates and the HMF concentration in the pretreated slurries were plotted against the calculated glucose:mannose ratios. The numbers for this plot is shown in table 1. As can be seen from figure 1, the glucose concentration in the enzymatic hydrolysate increases at higher glucose:mannose ratios as the severity of the pretreatment increases. However at glucose:mannose ratios above 1.5:1 the glucose concentration does not increase further. In fact the glucose levels even decreases. Furthermore, it is evident that the HMF concentrations starts to increase rapidly at glucose:mannose ratios above 1.5:1. This shows that the sugars are degraded into HMF which decreases the sugar yield. Furthermore, the concentrations of HMF and other inhibitors will lead to a lower yield in subsequent fermentation steps which are known to be inhibited by HMF and other fermentation inhibitors. The inventor further performed fermentation experiments which showed that the best ethanol yield was obtained when the glucose:mannose ratio was in the range 1:1 to 1.6:1 and even better yields were obtained in the range 1.2: 1 to 1.4 :1, data not shown. Finally the present inventor has also demonstrated that a continuous demo scale pretreatment process can be efficiently controlled by measuring the glucose : mannose ratio and controlling the severity of the pretreatment such that the ratio is kept within the predetermined reference value 1:1 to 1.6:1, preferably 1.2: 1 to 1.4 :1, data not shown.

**Table 1**

| Glucose/mannose (after pretreatment) | Glucose (g/l) after 24h Enz. Hydrolyz. | HMF g/l in pretreated slurry |
|---|---|---|
| 0,7 | 10,09 | 1.8 |
| 0,9 | 10,85 | 1.9 |
| 1,1 | 11,65 | 2.2 |
| 1,3 | 13,4 | 2.8 |
| 1,5 | 13,95 | 3.5 |
| 1,7 | 13,30 | 4.2 |
| 1,9 | 13,2 | 5.5 |
| 2,1 | 13,51 | 7.6 |

## Claims

1. Method for pretreating a softwood starting material comprising the following steps:
a) pretreating the softwood starting material in a pretreatment process to form a slurry;
b) measuring the concentration of glucose and the concentration of mannose in the slurry formed in step a);
c) calculating a ratio glucose : mannose from the concentrations measured in step b)
d) comparing the ratio calculated in step c) to a reference value and if the ratio calculated in step c) differs from the reference value, controlling at least one process parameter of the pretreatment process in response to the difference.

2. Method according to claim 1 further comprising the step e1) hydrolyzing the slurry obtained in step a) such that a sugar solution is obtained

3. Method according to claim 1 or 2 further comprising the step e2) anaerobic digestion of the slurry obtained in step a) or of the sugar solution obtained in step e1) such that biogas is obtained

4. Method according to claim 2 wherein the hydrolyzing in step e1) is enzymatic hydrolysis

5. Method according to any of the previous claims wherein:
- if the ratio glucose : mannose is lower than the reference value at least one process parameter of the pretreatment process is controlled such that the severity of the pretreatment is increased, and
- if the ratio glucose : mannose is higher than the reference value at least one process parameter of the pretreatment process is controlled such that the severity of the pretreatment is decreased

6. Method according to any of the previous claims wherein the ratio (w/w) glucose : mannose is calculated in step c) and the reference value is in the range 1:1 to 1.6:1.

7. Method according to any of the previous claims wherein the reference value is an interval

8. Method according to any of the previous claims wherein process parameter of the pretreatment process is controlled by:
- adjusting a temperature of the pretreatment step;
- adjusting a pressure of the pretreatment step
- adjusting a residence time of the pretreatment step and/or
- adjusting a pH of the pretreatment step

9. Method according to claim 8 wherein process parameter of the pretreatment process is controlled such that the severity of the pretreatment is increased by:
- increasing a temperature of the pretreatment step;
- increasing a pressure of the pretreatment step
- increasing a residence time of the pretreatment step and/or
- decreasing a pH of the pretreatment step if the pretreatment is acidic pretreatment or increasing a pH of the pretreatment step if the pretreatment is alkaline pretreatment

10. Method according to claims 8-9 wherein process parameter of the pretreatment process is controlled such that the severity of the pretreatment is decreased by:
- decreasing a temperature of the pretreatment step;
- decreasing a pressure of the pretreatment step
- decreasing a residence time of the pretreatment step and/or
- increasing a pH of the pretreatment step if the pretreatment is acidic pretreatment or decreasing a pH of the pretreatment step if the pretreatment is alkaline pretreatment

11. Method according to any of the previous claims wherein the pretreatment is acidic pretreatment using an acidic catalyst

12. Method according to claim 11 wherein the acidic catalyst is an acid solution or a gas.

13. Method according to claims 11-12 wherein the catalyst is selected from sulfuric acid, sulfurous acid and sulfuric acid

14. Method according to any of the previous claims wherein the pretreatment includes steam explosion

15. Method according to any of the previous claims wherein the pretreatment is a dilute acid pretreatment

## Patentansprüche

1. Verfahren zum Behandeln eines Weichholzausgangsmaterials mit den folgenden Schritten:
a) Vorbehandeln des Weichholzausgangsmaterials in einem Vorbehandlungsprozess zur Bildung einer Aufschlämmung;
b) Messen der Konzentration von Glucose und der Konzentration von Mannose in der in Schritt a) gebildeten Aufschlämmung;
c) aus den in Schritt b) gemessenen Konzentrationen Berechnen eines Glucose:Mannose-Verhältnisses,
d) Vergleichen des in Schritt c) berechneten Verhältnisses mit einem Bezugswert, und wenn das in Schritt c) berechnete Verhältnis von dem Bezugswert abweicht, Steuern von mindestens einem Prozessparameter des Vorbehandlungsprozesses als Reaktion auf die Abweichung.

2. Verfahren nach Anspruch 1, ferner umfassend den Schritt
e1) Hydrolysieren der in Schritt a) erhaltenen Aufschlämmung derart, dass eine Zuckerlösung erhalten wird.

3. Verfahren nach Anspruch 1 oder 2, ferner umfassend den Schritt
e2) anaerober Abbau der in Schritt a) erhaltenen Aufschlämmung oder der in Schritt e1) erhaltenen Zuckerlösung, sodass Biogas erhalten wird.

4. Verfahren nach Anspruch 2, wobei das Hydrolysieren in Schritt e1) eine enzymatische Hydrolyse ist.

5. Verfahren nach einem der vorhergehenden Ansprüche, wobei:
- wenn das Glucose:Mannose-Verhältnis kleiner als der Bezugswert ist, mindestens ein Prozessparameter des Vorbehandlungsprozesses derart gesteuert wird, dass die Intensität der Vorbehandlung erhöht wird, und
- wenn das Glucose:Mannose-Verhältnis größer als der Bezugswert ist, mindestens ein Prozessparameter des Vorbehandlungsprozesses derart gesteuert wird, dass die Intensität der Vorbehandlung gesenkt wird.

6. Verfahren nach einem der vorhergehenden Ansprüche, wobei das Glucose:Mannose-Verhältnis (m/m) in Schritt c) berechnet wird und der Bezugswert im Bereich 1:1 bis 1,6:1 liegt.

7. Verfahren nach einem der vorhergehenden Ansprüche, wobei der Bezugswert ein Intervall ist.

8. Verfahren nach einem der vorhergehenden Ansprüche, wobei ein Prozessparameter des Vorbehandlungsprozesses gesteuert wird durch:
- Einstellen einer Temperatur des Vorbehandlungsschritts;
- Einstellen eines Drucks des Vorbehandlungsschritts
- Einstellen einer Verweilzeit des Vorbehandlungsschritts, und/oder
- Einstellen eines pH-Werts des Vorbehandlungsschritts.

9. Verfahren nach Anspruch 8, wobei ein Prozessparameter des Vorbehandlungsprozesses derart gesteuert wird, dass die Intensität der Vorbehandlung erhöht wird durch:
- Erhöhen einer Temperatur des Vorbehandlungsschritts;
- Erhöhen eines Drucks des Vorbehandlungsschritts
- Verlängern einer Verweilzeit des Vorbehandlungsschritts und/oder
- Senken eines pH-Werts des Vorbehandlungsschritts, wenn die Vorbehandlung eine saure Vorbehandlung ist, oder Erhöhen eines pH-Werts des Vorbehandlungsschritts, wenn die Vorbehandlung eine alkalische Vorbehandlung ist.

10. Verfahren nach Anspruch 8 bis 9, wobei ein Prozessparameter des Vorbehandlungsprozesses derart gesteuert wird, dass die Intensität der Vorbehandlung gesenkt wird durch:
- Senken einer Temperatur des Vorbehandlungsschritts;
- Senken eines Drucks des Vorbehandlungsschritts
- Verkürzen einer Verweilzeit des Vorbehandlungsschritts und/oder
- Erhöhen eines pH-Werts des Vorbehandlungsschritts, wenn die Vorbehandlung eine saure Vorbehandlung ist, oder Senken eines pH-Werts des Vorbehandlungsschritts, wenn die Vorbehandlung eine alkalische Vorbehandlung ist.

11. Verfahren nach einem der vorhergehenden Ansprüche, wobei die Vorbehandlung eine saure Vorbehandlung unter Verwendung eines sauren Katalysators ist.

12. Verfahren nach Anspruch 11, wobei der saure Katalysator eine Säurelösung oder ein Gas ist.

13. Verfahren nach Anspruch 11 bis 12, wobei der Katalysator aus Schwefelsäure, schwefliger Säure und Schwefelsäure ausgewählt wird.

14. Verfahren nach einem der vorhergehenden Ansprüche, wobei die Vorbehandlung eine Dampfexplosion umfasst.

15. Verfahren nach einem der vorhergehenden Ansprüche, wobei die Vorbehandlung eine Vorbehandlung mit einer verdünnten Säure ist.

## Revendications

1. Procédé de prétraitement d'un matériau de départ de bois tendre comprenant les étapes suivantes :
a) prétraiter le matériau de départ de bois tendre dans un processus de prétraitement pour former une solution épaisse ;
b) mesurer la concentration de glucose et la concentration de mannose dans la solution épaisse formée dans l'étape a) ;
c) calculer un rapport glucose : mannose à partir des concentrations mesurées dans l'étape b)
d) comparer le rapport calculé dans l'étape c) à une valeur de référence et si le rapport calculé dans l'étape c) diffère de la valeur de référence, contrôler au moins un paramètre de processus du processus de prétraitement en réponse à la différence.

2. Procédé selon la revendication 1 comprenant en outre l'étape
e1) d'hydrolyser la solution épaisse obtenue dans l'étape a) de telle manière qu'une solution de sucre est obtenue.

3. Procédé selon la revendication 1 ou 2 comprenant en outre l'étape
e2) de digestion anaérobie de la solution épaisse obtenue dans l'étape a) ou de la solution de sucre obtenue dans l'étape e1) telle qu'un biogaz est obtenu.

4. Procédé selon la revendication 2 dans lequel l'hydrolyse dans l'étape e1) est une hydrolyse enzymatique.

5. Procédé selon l'une quelconque des revendications précédentes dans lequel :
- si le rapport glucose : mannose est inférieur à la valeur de référence au moins un paramètre de processus du processus de prétraitement est contrôlé de telle manière que la sévérité du prétraitement est augmentée, et
- si le rapport glucose : mannose est supérieur à la valeur de référence au moins un paramètre de processus du processus de prétraitement est contrôlé de telle manière que la sévérité du prétraitement est diminuée.

6. Procédé selon l'une quelconque des revendications précédentes dans lequel le rapport (p/p) glucose : mannose est calculé dans l'étape c) et la valeur de référence se situe dans la plage de 1 : 1 à 1,6 : 1.

7. Procédé selon l'une quelconque des revendications précédentes dans lequel la valeur de référence est un intervalle.

8. Procédé selon l'une quelconque des revendications précédentes dans lequel le paramètre de processus du processus de prétraitement est contrôlé par :
- l'ajustement d'une température de l'étape de prétraitement ;
- l'ajustement d'une pression de l'étape de prétraitement
- l'ajustement d'un temps de résidence de l'étape de prétraitement et/ou
- l'ajustement d'un pH de l'étape de prétraitement.

9. Procédé selon la revendication 8 dans lequel un paramètre de processus du processus de prétraitement est contrôlé de telle manière que la sévérité du prétraitement est augmentée par :
- l'augmentation d'une température de l'étape de prétraitement ;
- l'augmentation d'une pression de l'étape de prétraitement
- l'augmentation d'un temps de résidence de l'étape de prétraitement et/ou
- la diminution d'un pH de l'étape de prétraitement si le prétraitement est un prétraitement acide ou l'augmentation d'un pH de l'étape de prétraitement si le prétraitement est un prétraitement alcalin.

10. Procédé selon les revendications 8 à 9 dans lequel un paramètre de processus du processus de prétraitement est contrôlé de telle manière que la sévérité du prétraitement est diminuée par :
- la diminution d'une température de l'étape de prétraitement ;
- la diminution d'une pression de l'étape de prétraitement
- la diminution d'une temps de résidence de l'étape de prétraitement et/ou
- l'augmentation d'un pH de l'étape de prétraitement si le prétraitement est un prétraitement acide ou la diminution d'un pH de l'étape de prétraitement si le prétraitement est un prétraitement alcalin.

11. Procédé selon l'une quelconque des revendications précédentes dan lequel le prétraitement est un prétraitement acide utilisant un catalyseur acide.

12. Procédé selon la revendication 11 dans lequel le catalyseur acide est une solution ou un gaz acide.

13. Procédé selon les revendications 11 à 12 dans lequel le catalyseur est choisi parmi l'acide sulfurique, l'acide sulfureux et l'acide sulfurique.

14. Procédé selon l'une quelconque des revendications précédentes dans lequel le prétraitement comprend une explosion de vapeur.

15. Procédé selon l'une quelconque des revendications précédentes dans lequel le prétraitement est un prétraitement acide dilué.
